(19) Europäisches Patentamt
European Patent Office
Office européen des brevets

(11) EP 3 290 038 A1

(12) **EUROPEAN PATENT APPLICATION**
published in accordance with Art. 153(4) EPC

(43) Date of publication:
**07.03.2018 Bulletin 2018/10**

(21) Application number: **16786600.3**

(22) Date of filing: **28.04.2016**

(51) Int Cl.:
***A61K 31/513*** *(2006.01)* ***A61P 1/00*** *(2006.01)*
***A61P 43/00*** *(2006.01)* ***A61K 31/4412*** *(2006.01)*
***A61K 31/519*** *(2006.01)* ***A61K 31/53*** *(2006.01)*
***A61P 35/00*** *(2006.01)*

(86) International application number:
**PCT/JP2016/063494**

(87) International publication number:
**WO 2016/175324 (03.11.2016 Gazette 2016/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **30.04.2015 JP 2015093305**

(71) Applicant: **Taiho Pharmaceutical Co., Ltd.**
**Chiyoda-ku**
**Tokyo 101-8444 (JP)**

(72) Inventor: **WAKASA, Takeshi**
**Tsukuba-shi**
**Ibaraki 300-2611 (JP)**

(74) Representative: **Schiener, Jens**
**Wächtershäuser & Hartz**
**Patentanwaltspartnerschaft mbB**
**Weinstraße 8**
**80333 München (DE)**

# (54) AGENT FOR ALLEVIATING ADVERSE REACTION TO ANTITUMOR DRUG

(57) Provided is an agent for alleviating side effect of antitumor drug, the agent alleviating side effect of antitumor drug when used in combination with the antitumor drug. Disclosed is an agent for alleviating side effect of antitumor drug, the agent containing a uracil compound represented by the following Formula (I) or a pharmacologically acceptable salt thereof as an active ingredient:

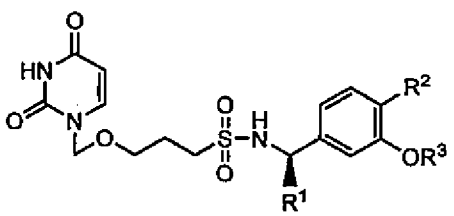

( I )

wherein $R^1$ represents a hydrogen atom or a C1-6 alkyl group; $R^2$ represents a hydrogen atom or a halogen atom; and $R^3$ represents a C1-6 alkyl group, a C2-6 alkenyl group, a C3-6 cycloalkyl group, a (C3-6 cycloalkyl) -C1-6 alkyl group, a halogeno-C1-6 alkyl group, or a saturated heterocyclic group.

Figure 1

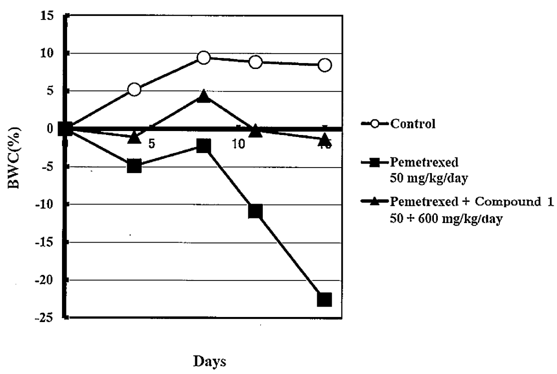


EP 3 290 038 A1

## Description

### Field of the Invention

The present invention relates to an agent for alleviating side effect of antitumor drug.

### Background of the Invention

Deoxyuridine triphosphatase (hereinafter, also referred to as "dUTPase" (EC3.6.1.23)) is a prophylactic DNA repair enzyme. This enzyme is an enzyme which specifically recognizes deoxyuridine triphosphate only among naturally-occurring nucleoside triphosphates and decomposes deoxyuridine triphosphate into deoxyuridine monophosphate and pyrophosphoric acid, and the enzyme is known to be essential for the survival of cells in both prokaryotes and eukaryotes.

In regard to malignant tumors, a correlation between the degree of malignancy and the amount of expression of dUTPase was observed (Non-Patent Documents 1 and 2), and it has been reported that a tumor with elevated expression of dUTPase exhibits tolerance to chemotherapy (Non-Patent Document 3). Furthermore, in cultured cancer cells, it has been shown that when the amount of expression of dUTPase is decreased by using siRNA, the antitumor effect of thymidylate synthase inhibitors (hereinafter, "TS inhibitors") is potentiated (Non-Patent Document 4).

Meanwhile, Patent Document 1 discloses uracil compounds having dUTPase inhibitory activity, and Patent Document 2 discloses that the same uracil compounds potentiate antitumor effects.

However, nothing has been known that the above-mentioned uracil compounds alleviate side effect of antitumor drugs.

### Citation List

#### Patent Document

Patent Document 1: WO 2009/147843
Patent Document 2: WO 2011/065541

#### Non Patent Document

Non-Patent Document 1: J Clin Pathol, 2009 Apr; 62(4):364-9
Non-Patent Document 2: Int J Cancer, 1999 Dec 22; 84(6):614-7
Non-Patent Document 3: Cancer Res, 2000 Jul 1; 60(13):3493-503
Non-Patent Document 4: Mol Pharmacol, 2004 Sep; 66(3):620-6

### Summary of the Invention

#### Problems to be solved by the Invention

An object of the present invention is to provide an agent for alleviating side effect of antitumor drug, the agent alleviating side effect of antitumor drug when used in combination with the antitumor drug.

#### Means for solving the Problem

The inventors of the present invention repeatedly conducted thorough investigations in order to solve the problem described above, and as a result, the inventors found that a uracil compound having a sulfonamide structure at the N-1-position of a uracil ring represented by the following Formula (I), or a salt thereof, has excellent side effect alleviating activity against an antitumor drug, and particularly gastrointestinal toxicity alleviating activity, and the uracil compound or a salt thereof is useful as an agent for alleviating side effect of the antitumor drug. Thus, the inventors completed the present invention.

Accordingly, the present invention provides the following [1] to [20].

[1] An agent for alleviating side effect of antitumor drug, the agent including, as an active ingredient, a uracil compound

represented by the following Formula (I) or a pharmacologically acceptable salt thereof:

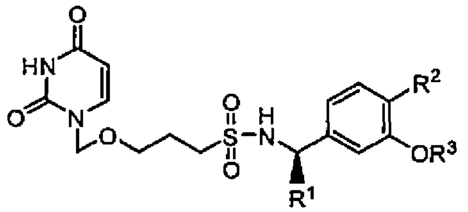

( I )

wherein $R^1$ represents a hydrogen atom or a C1-6 alkyl group; $R^2$ represents a hydrogen atom or a halogen atom; and $R^3$ represents a C1-6 alkyl group, a C2-6 alkenyl group, a C3-6 cycloalkyl group, a (C3-6 cycloalkyl) -C1-6 alkyl group, a halogeno-C1-6 alkyl group, or a saturated heterocyclic group.

[2] The agent for alleviating side effect of antitumor drug according to [1], wherein the uracil compound is a compound represented by Formula (I), in which $R^1$ represents a hydrogen atom, a methyl group, or an ethyl group; $R^2$ represents a hydrogen atom; and $R^3$ represents a cyclopentyl group, a cyclopropylmethyl group, or a 2,2,2-trifluoroethyl group.

[3] The agent for alleviating side effect of antitumor drug according to [1] or [2], wherein the uracil compound is selected from the group of the following compounds:

N-(1-(3-(cyclopentyloxy)phenyl)ethyl)-3-((2,4-dioxo-3,4-dihydropyr imidin-1(2H)-yl)methoxy)propane-1-sulfonamide,

N-(3-(cyclopropylmethoxy)benzyl)-3-((2,4-dioxo-3,4-dihydropyrimidi n-1(2H)-yl)methoxy)propane-1-sulfonamide, and

(R)-N-(1-(3-(2,2,2-trifluoroethoxy)phenyl)ethyl)-3--((2,4-dioxo-3,4 -dihydropyrimidin-1(2H)-yl)methoxy)propane-1-sulfonamide.

[4] The agent for alleviating side effect of antitumor drug according to any one of [1] to [3], wherein the antitumor drug is an antimetabolite.

[5] The agent for alleviating side effect of antitumor drug according to any one of [1] to [3], wherein the antitumor drug is any one of 5-fluorouracil (5-FU), a tegafur/gimeracil/oteracil potassium combination drug (tegafur : gimeracil : oteracil = 1 : 0.4 : 1 (molar ratio)), and pemetrexed.

[6] Use of a uracil compound represented by the following Formula (I) or a pharmacologically acceptable salt thereof, for the production of an agent for alleviating side effect of antitumor drug:

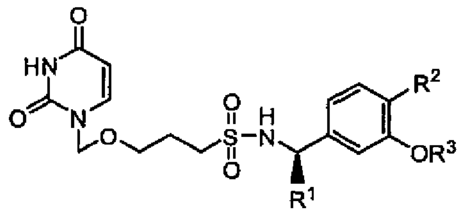

( I )

wherein $R^1$ represents a hydrogen atom or a C1-6 alkyl group; $R^2$ represents a hydrogen atom or a halogen atom; and $R^3$ represents a C1-6 alkyl group, a C2-6 alkenyl group, a C3-6 cycloalkyl group, a (C3-6 cycloalkyl) -C1-6 alkyl group, a halogeno-C1-6 alkyl group, or a saturated heterocyclic group.

[7] The use according to [6], wherein the uracil compound is a compound represented by Formula (I), in which $R^1$ represents a hydrogen atom, a methyl group, or an ethyl group; $R^2$ represents a hydrogen atom; and $R^3$ represents a cyclopentyl group, a cyclopropylmethyl group, or a 2,2,2-trifluoroethyl group.

[8] The use according to [6], wherein the uracil compound is a compound selected from the group consisting of the following compounds:

N-(1-(3-(cyclopentyloxy)phenyl)ethyl)-3-((2,4-dioxo-3,4-dihydropyr imidin-1(2H)-yl)methoxy)propane-1-sulfonamide,

N-(3-(cyclopropylmethoxy)benzyl)-3-((2,4-dioxo-3,4-dihydropyrimidi n-1(2H)-yl)methoxy)propane-1-sulfonamide, and

(R)-N-(1-(3-(2,2,2-trifluoroethoxy)phenyl)ethyl)--3-((2,4-dioxo-3,4 -dihydropyrimidin-1(2H)-yl)methoxy)propane-1-sulfonamide.

[9] The use according to any one of [6] to [8], wherein the antitumor drug is an antimetabolite.
[10] The use according to any one of [6] to [8], wherein the antitumor drug is any one of 5-fluorouracil (5-FU), a tegafur/gimeracil/oteracil potassium combination drug (tegafur : gimeracil : oteracil = 1 : 0.4 : 1 (molar ratio)), and pemetrexed.
[11] A uracil compound represented by the following Formula (I) or apharmacologically acceptable salt thereof, for use in the alleviation of side effect of antitumor drug:

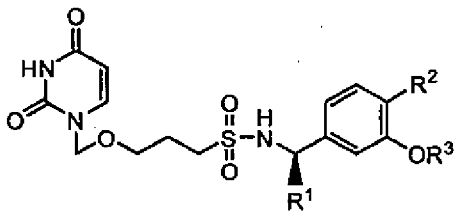

(I)

wherein $R^1$ represents a hydrogen atom or a C1-6 alkyl group; $R^2$ represents a hydrogen atom or a halogen atom; and $R^3$ represents a C1-6 alkyl group, a C2-6 alkenyl group, a C3-6 cycloalkyl group, a (C3-6 cycloalkyl) -C1-6 alkyl group, a halogeno-C1-6 alkyl group, or a saturated heterocyclic group.
[12] The uracil compound or a pharmacologically acceptable salt thereof according to [11], wherein in Formula (I), $R^1$ represents a hydrogen atom, a methyl group, or an ethyl group; $R^2$ represents a hydrogen atom; and $R^3$ represents a cyclopentyl group, a cyclopropylmethyl group, or a 2,2,2-trifluoroethyl group.
[13] The uracil compound or a pharmacologically acceptable salt thereof according to [11], wherein the uracil compound or the salt is a compound selected from the group consisting of the following compounds, or a pharmacologically acceptable salt thereof:

N-(1-(3-(cyclopentyloxy)phenyl)ethyl)-3-((2,4-dioxo-3,4-dihydropyr imidin-1(2H)-yl)methoxy)propane-1-sulfonamide,

N-(3-(cyclopropylmethoxy)benzyl)-3-((2,4-dioxo-3,4-dihydropyrimidi n-1(2H)-yl)methoxy)propane-1-sulfonamide, and

(R)-N-(1-(3-(2,2,2-trifluoroethoxy)phenyl)ethyl)-3-((2,4-dioxo-3,4 -dihydropyrimidin-1(2H)-yl)methoxy)propane-1-sulfonamide.

[14] The uracil compound or a pharmacologically acceptable salt thereof according to any one of [11] to [13], wherein the antitumor drug is an antimetabolite.
[15] The uracil compound or a pharmacologically acceptable salt thereof according to any one of [11] to [13], wherein the antitumor drug is any one of 5-fluorouracil (5-FU), a tegafur/gimeracil/oteracil potassium combination drug (tegafur : gimeracil : oteracil = 1 : 0.4 : 1 (molar ratio)), and pemetrexed.
[16] A method for alleviating side effect of antitumor drug, the method including administering an effective amount of a uracil compound represented by the following Formula (I) or a pharmacologically acceptable salt thereof to an object in need of the alleviation of side effect of antitumor drug:

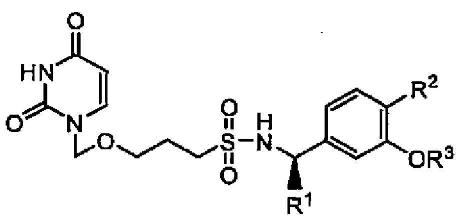

(I)

wherein $R^1$ represents a hydrogen atom or a C1-6 alkyl group; $R^2$ represents a hydrogen atom or a halogen atom; and $R^3$ represents a C1-6 alkyl group, a C2-6 alkenyl group, a C3-6 cycloalkyl group, a (C3-6 cycloalkyl) -C1-6 alkyl group, a halogeno-C1-6 alkyl group, or a saturated heterocyclic group.

[17] The method according to [16], wherein the uracil compound is a compound represented by Formula (I), in which $R^1$ represents a hydrogen atom, a methyl group, or an ethyl group; $R^2$ represents a hydrogen atom; and $R^3$ represents a cyclopentyl group, a cyclopropylmethyl group, or a 2,2,2-trifluoroethyl group.

[18] The method according to [16], wherein the uracil compound is selected from the group consisting of the following compounds:

N-(1-(3-(cyclopentyloxy)phenyl)ethyl)-3-((2,4--dioxo-3,4-dihydropyr imidin-1(2H)-yl)methoxy)propane-1-sulfonamide,

N-(3-(cyclopropylmethoxy)benzyl)-3-((2,4-dioxo-3,4-dihydropyrimidi n-1(2H)-yl)methoxy)propane-1-sulfonamide, and

(R)-N-(1-(3-(2,2,2-trifluoroethoxy)phenyl)ethyl)-3-((2,4-dioxo-3,4 -dihydropyrimidin-1(2H)-yl)methoxy)propane-1-sulfonamide.

[19] The method according to any one of [16] to [18], wherein the antitumor drug is an antimetabolite.

[20] The method according to any one of [16] to [18], wherein the antitumor drug is any one of 5-fluorouracil (5-FU), a tegafur/gimeracil/oteracil potassium combination drug (tegafur : gimeracil : oteracil = 1 : 0.4 : 1 (molar ratio)), and pemetrexed.

Effects of the Invention

When the agent for alleviating side effect of antitumor drug of the present invention is used in combination with an antitumor drug, side effects of an antitumor drug, such as gastrointestinal toxicity, canbe alleviated. Therefore, according to the present invention, cancer chemotherapy with higher safety can be provided, and since it is also possible to administer an increased amount of an antitumor drug, cancer chemotherapy with higher therapeutic effects can be provided.

Brief Description of Drawings

Fig. 1 is a diagram illustrating the rate of body weight change by using pemetrexed in a tumor-bearing mouse.
Fig. 2 is a diagram illustrating the diarrhea incidence by using pemetrexed in a tumor-bearing mouse.
Fig. 3 is a diagram illustrating the rate of body weight change by using S-1 in a tumor-bearing mouse.
Fig. 4 is a diagram illustrating the rate of body weight change by using S-1 in a tumor-bearing mouse.
Fig. 5 is a diagram illustrating the diarrhea incidence by using S-1 in a tumor-bearing mouse.
Fig. 6 is a diagram illustrating the rate of body weight change by using S-1 in a non-tumor-bearing mouse.
Fig. 7 is a diagram illustrating the diarrhea incidence by using S-1 in a non-tumor-bearing mouse.
Fig. 8 is a diagram illustrating the rate of body weight change by using FCD in a non-tumor-bearing mouse.
Fig. 9 is a diagram illustrating the diarrhea incidence by using FCD in a non-tumor-bearing mouse.

Detailed Description of the Invention

In Formula (I), the "C1-6 alkyl group" represented by $R^1$ may be a linear or branched hydrocarbon group having 1 to 6 carbon atoms, and specific examples include a methyl group, an ethyl group, a n-propyl group, an isopropyl group, a n-butyl group, a sec-butyl group, a tert-butyl group, a n-pentyl group, and a n-hexyl group. A C1-3 alkyl group is preferred, and a methyl group and an ethyl group are more preferred.

In Formula (I), examples of the "halogen atom" represented by $R^2$ include a fluorine atom, a chlorine atom, a bromine atom, and an iodine atom, and a fluorine atom is preferred.

In Formula (I), $R^2$ is preferably a hydrogen atom.

In Formula (I), examples of the "C1-6 alkyl group" represented by $R^3$ include the same groups as those mentioned above for $R^1$.

In Formula (I), the "C2-6 alkenyl group" represented by $R^3$ represents a hydrocarbon group having 2 to 6 carbon atoms and containing a carbon-carbon double bond, and examples include a vinyl group, an allyl group, a methylvinyl

group, a propenyl group, a butenyl group, a pentenyl group, and a hexenyl group.

In Formula (I), examples of the "C3-6 cycloalkyl group" represented by $R^3$ include a cyclopropyl group, a cyclobutyl group, a cyclopentyl group, and a cyclohexyl group, and a preferred example is a cyclopentyl group.

In Formula (I), the "(C3-6 cycloalkyl)-C1-6 alkyl group" represented by $R^3$ represents an alkyl group having 1 to 6 carbon atoms and having the above-mentioned cycloalkyl group, and a preferred example is a cyclopropylmethyl group.

In Formula (I), the "halogeno-C1-6 alkyl group" represented by $R^3$ represents an alkyl group having 1 to 6 carbon atoms and having the above-mentioned halogen atom, and a preferred example is a 2,2,2-trifluoroethyl group.

In Formula (I), the "saturated heterocyclic group" represented by $R^3$ represents a monocyclic or bicyclic saturated heterocyclic group preferably having one or two of any atom preferably selected from an oxygen atom, a nitrogen atom, and a sulfur atom. Examples include a pyrrolidinyl group, a piperidinyl group, a piperazinyl group, a hexamethyleneimino group, a morpholino group, a thiomorpholino group, a homopiperidinyl group, a tetrahydrofuryl group, and a tetrahydropyryl group.

$R^3$ is preferably a cyclopentyl group, a cyclopropylmethyl group, or a 2,2,2-trifluoroethyl group.

In Formula (I), $R^1$ represents a hydrogen atom, a methyl group, or an ethyl group; $R^2$ represents a hydrogen atom; and $R^3$ represents a cyclopentyl group, a cyclopropylmethyl group, or a 2,2,2-trifluoroethyl group, is preferred.

Examples of the pharmacologically acceptable salt of the uracil compound represented by Formula (I) include acid addition salts with inorganic acids such as hydrochloric acid, hydrobromica cid, hydroiodic acid, sulfuric acid, nitric acid, and phosphoric acid, or with organic acids such as formic acid, acetic acid, propionic acid, oxalic acid, malonic acid, succinic acid, fumaric acid, maleic acid, lactic acid, malic acid, citric acid, tartaric acid, carbonic acid, picric acid, methanesulfonic acid, para-toluenesulfonic acid, and glutamic acid; salts with inorganic bases such as sodium, potassium, magnesium, calcium, and aluminum, with organic bases such as methylamine, ethylamine, meglumine, and ethanolamine, or with basic amino acids such as lysine, arginine, and ornithine; and ammonium salts. Furthermore, the uracil compound represented by Formula (I) also includes optical isomers as well as hydrates.

The uracil compound represented by Formula (I) of the present invention can be produced according to Patent Document 1 or Patent Document 2.

As described in the Examples given below, the uracil compound represented by Formula (I) has an effect of alleviating side effects of various antitumor drugs when used in combination with those antitumor drugs.

Therefore, the uracil compound represented by Formula (I) or a pharmacologically acceptable salt thereof can be used as an agent for alleviating side effect of antitumor drug.

Furthermore, the uracil compound represented by Formula (I) or a pharmacologically acceptable salt thereof can be used in order to promote alleviation of side effect of antitumor drug by administering an effective amount of the uracil compound or a salt thereof.

The uracil compound represented by Formula (I) or a pharmacologically acceptable salt thereof can also be used in order to produce an agent for alleviating side effect of antitumor drug.

According to the present invention, "alleviation of side effect of antitumor drug" means that in a case in which the uracil compound represented by Formula (I) or a pharmacologically acceptable salt thereof is used in combination with an antitumor drug, the uracil compound or a salt thereof alleviates side effect caused by said antitumor drug.

In the present invention, the "side effect" may be a symptom presented as normal cells such as hematopoietic cells, oral mucosa, gastrointestinal mucosa, and hair root cells are injured by an antitumor drug. Examples of the side effect include symptoms such as anemia, infectious diseases, and bleeding caused by the hematopoietic cells being injured; hair loss caused by the hair root cells being injured; mouth ulcer caused by the oral mucosa being injured; and vomiting and diarrhea caused by the gastrointestinal mucosa being injured (gastrointestinal toxicity). Among these, the agent of the present invention is more effective against gastrointestinal toxicity, and is particularly effective against diarrhea.

The antitumor drug, of which side effect is alleviated by the agent for alleviating side effect of antitumor drug of the present invention, are not particularly limited, examples of such antitumor drug include alkylating agents such as cyclophosphamide and nimustine; platinum preparations such as cisplatin, carboplatin, and oxaliplatin; antimetabolites; and plant alkaloid-based antitumor drugs such as paclitaxel, docetaxel, and irinotecan. A preferred antitumor drug is an antimetabolite.

An antimetabolite as used herein is a compound having a chemical structure similar to that of a substance that serve as a material for nucleic acids when cancer cells undergo mitosis/proliferation, or a drug containing the compound as an active ingredient, and the antimetabolite refers to an anticancer agent that interrupts the biosynthesis of nucleic acids or the biosynthetic pathway of nucleic acids and suppresses proliferation. Examples thereof include pyrimidine-based antimetabolites such as 5-fluorouracil (5-FU), a tegafur/gimeracil/oteracil potassium combination drug (tegafur : gimeracil : oteracil = 1 : 0.4 : 1 (molar ratio) ; hereinafter, "S-1"), a tegafur/uracil combination drug (tegafur : uracil = 1 : 4; hereinafter, "UFT"), capecitabine, doxifluridine, 5-fluoro-2'-deoxyuridine (FdUrd), gemcitabine, and citarabine; purine-based antimetabolites such as fludarabine, cladribine, and nelarabine; and folate antimetabolites such as pemetrexed

and methotrexate.

Among these, a thymidylate (TMP) synthetic pathway inhibitor is preferred. A thymidylate synthetic pathway inhibitor refers to a compound that directly or indirectly inhibits an enzyme related to the biosynthesis of TMP, represented by a thymidylate synthase inhibitor or a dihydrofolate reductase inhibitor among antimetabolites, or a drug containing the compound as an active ingredient. A thymidylate synthase inhibitor refers to a compound that inhibits a thymidylate synthase, or a drug containing the enzyme as an active ingredient, and examples include fluorinated pyrimidine-based antimetabolites such as 5-fluorouracil (5-FU), S-1, UFT, capecitabine, doxifluridine, 5-fluoro-2'-deoxyuridine (FdUrd), and carmofur (yamafur); folate antimetabolites such as pemetrexed, methotrexate, and raltitrexed; and noltrexed dihydrochloride. A dihydrofolate reductase inhibitor refers to a compound inhibiting an enzyme that biosynthesizes tetrahydrofolate, which is essential for de novo synthesis of purines or thymidylic acid, or a drug containing the compound as an active ingredient. Examples include antifolates such as pralatrexate and edatrexate; pyrimethamine, brodimoprim, and trimetrexate glucuronate.

The antitumor drug, of which side effect is alleviated by the agent for alleviating side effect of antitumor drug of the present invention, is more preferably a thymidylate synthase inhibitor; even more preferably a drug containing tegafur and gimeracil as active ingredients, or pemetrexed; and particularly preferably S-1 or pemetrexed.

Furthermore, there are no particular limitations on malignant tumors that can be treated when the agent for alleviating side effect of antitumor drug of the present invention is used in combination with the above-mentioned antitumor drug; however, examples include head and neck cancer, esophageal cancer, stomach cancer, colon cancer, rectal cancer, liver cancer, gall bladder/bile duct cancer, pancreatic cancer, lung cancer, breast cancer, ovarian cancer, cervical cancer, uterine cancer, renal cancer, urinary bladder cancer, prostate cancer, testicular tumor, bone/soft tissue sarcoma, leukemia, malignant lymphoma, multiple myeloma, skin cancer, and brain tumor.

When the agent for alleviating side effect of antitumor drug of the present invention is mixed with an antitumor drug and used, the agent enables provision of cancer chemotherapy with alleviated side effect of antitumor drug. In this case, the agent for alleviating side effect of antitumor drug of the present invention may be prepared into a single-agent type formulation including an antitumor drug, or the agent for alleviating side effect of antitumor drug of the present invention may also be prepared into a form that is formulated separately from an antitumor drug and used in combination with the antitumor drug at the time of use.

In the case of using the agent for alleviating side effect of antitumor drug of the present invention and an antitumor drug as separate formulations, the means for administration of the agent for alleviating side effect of antitumor drug of the present invention and the means for administration of the antitumor drug may be identical to each other, or may be different from each other (for example, oral administration and injection) . Also, the timing for administration of the agent for alleviating side effect of antitumor drug and the antitumor drug may be such that the two agents may be administration simultaneously or separately at a certain time interval. The order of administration of the two agents is also not limited and can be selected as appropriate. Preferably, the agents maybe administered simultaneously, or one agent may be administered first, followed by administration of the other agent after 6 hours.

In the case of using the agent for alleviating side effect of antitumor drug of the present invention and antitumor drug as separate formulations, the agents can be provided in the form of a "kit". The agent for alleviating side effect of antitumor drug of the present invention and an antitumor drug may be prepared respectively into a certain preferable formulations, and may be packaged into various containers that are conventionally used according to the formulation, and thereby a kit for cancer treatment for mammals including human being can be prepared.

The agent for alleviating side effect of antitumor drug of the present invention is used as a medicine (pharmaceutical composition), and in this case, a pharmacological carrier can be incorporated into the pharmaceutical composition as necessary, in addition to the uracil compound represented by Formula (I) or a pharmacologically acceptable salt thereof, so that the pharmaceutical composition can be prepared into various dosage forms of administration according to the prophylactic or therapeutic purpose.

Examples of the dosage forms include an oral preparation, an injectable preparation, a suppository, an ointment, and a transdermal patch, and an oral preparation is preferred. These dosage forms can be respectively produced by formulation methods that are conventionally known to those ordinarily skilled in the art.

Regarding the pharmacological carrier, various conventionally used organic or inorganic carrier substances are used as preparation materials, and the carrier substances are incorporated as, for example, an excipient, a binder, a disintegrant, a lubricating agent, and a coloring agent for solid preparations; and as a solvent, a dissolution aid, a suspending agent, an isotonizing agent, a buffering agent, and a soothing agent for liquid preparations. Furthermore, if necessary, preparation additives such as an antiseptic agent, an antioxidant, a coloring agent, a sweetening agent, and a stabilizer can also be used.

In the case of preparing an oral solid preparation, for example, an excipient, and if necessary, a binder, a disintegrant, a lubricating agent, a coloring agent, and a corrigent are added to the uracil compound represented by Formula (I) or a pharmacologically acceptable salt thereof, and then, for example, a tablet preparation, a coated tablet preparation, a granular preparation, a powder preparation, or a capsule preparation can be produced according to routine

procedures.

In the present invention, examples of the excipient include lactose, sucrose, D-mannitol, glucose, starch, calcium carbonate, kaolin, microcrystalline cellulose, and silicic anhydride. Examples of the binder include water, ethanol, 1-propanol, 2-propanol, simple syrup, a glucose solution, an $\alpha$-starch solution, a gelatin solution, D-mannitol, carboxymethyl cellulose, hydroxypropyl cellulose, hydropropyl starch, methyl cellulose, ethyl cellulose, shellac, calcium phosphate, and polyvinylpyrrolidone. Examples of the disintegrant include dried starch, sodium alginate, powdered agar, sodium hydrogen carbonate, calcium carbonate, sodium lauryl sulfate, stearic acid monoglyceride, and lactose. Examples of the lubricating agent include purified talc, sodium stearate, magnesium stearate, borax, and polyethylene glycol. Examples of the coloring agent include titanium oxide and iron oxide. Examples of the corrigent include sucrose, orange peel, citric acid, and tartaric acid.

In the case of preparing an oral liquid preparation, for example, a corrigent, a buffering agent, a stabilizer, and a flavoring agent may be added to the uracil compound represented by Formula (I) or a pharmacologically acceptable salt thereof, and for example, an internal liquid medicine, a syrup preparation, or an elixir can be produced by a conventional method. In this case, the substances mentioned above may be favorably used as the corrigent, and examples of the buffering agent include sodium citrate, while examples of the stabilizer include tragacanth, gum arabic, and gelatin. If necessary, the oral preparation may also be provided with an enteric coating, or for the purpose of sustaining the effects, may be provided with a coating by means of a known method. Examples of such a coating agent include hydroxypropylmethyl cellulose, ethyl cellulose, hydroxymethyl cellulose, hydroxypropyl cellulose, polyoxyethylene glycol, and TWEEN 80 (registered trademark).

In the case of producing an injectable preparation, for example, a pH adjusting agent, a buffering agent, a stabilizer, an isotonizing agent, and a local anesthetic agent may be added to the uracil compound represented by Formula (I) or a pharmacologically acceptable salt thereof, and a subcutaneous, intramuscular, and intravenous injectable preparations can be prepared by routine procedures. Examples of the pH adjusting agent and buffering agent in this case include sodium citrate, sodium acetate, and sodium phosphate. Examples of the stabilizer include sodium pyrosulfite, EDTA, thioglycolic acid, and thiolactic acid. Examples of the local anesthetic agent include procaine hydrochloride and lidocaine hydrochloride. Examples of the isotonizing agent include sodium chloride, glucose, D-mannitol, and glycerin.

In the case of preparing a suppository, a carrier for formulation known in the pertinent field, for example, polyethylene glycol, lanolin, cacao fats, or fatty acid triglycerides, and if necessary, for example, a surfactant such as TWEEN 80 (registered trademark) may be added to the uracil compound represented by Formula (I) or a pharmacologically acceptable salt thereof, and then a suppository can be produced by a routine procedure.

In the case of preparing an ointment, for example, a base, a stabilizer, a wetting agent, and a preservative that are conventionally used are mixed as necessary with the uracil compound represented by Formula (I) or a pharmacologically acceptable salt thereof, and the ointment is mixed and formulated by a routine procedure. Examples of the base include liquidparaffin, white petrolatum, white beeswax, octyl dodecyl alcohol, and paraffin. Examples of the preservative include methyl paraoxybenzoate, ethyl paraoxybenzoate, and propyl paraoxybenzoate.

In the case of preparing a transdermal patch, for example, the ointment, cream, gel, and paste described above may be applied on a conventional support by a routine procedure. Suitable examples of the support include woven fabrics or non-woven fabrics formed from cotton, staple fibers, and chemical fibers; and films or foamed sheets made of, for example, soft vinyl chloride, polyethylene, and polyurethane.

The amount of the uracil compound represented by Formula (I) or a pharmacologically acceptable salt thereof to be incorporated in the various unit dosage forms mentioned above may vary depending on the symptoms of the patient to whom this compound or salt should be applied, or depending on the formulation, the amount is generally about 0.05 to 1,000 mg in an oral preparation, about 0.01 to 500 mg in an injectable preparation, and about 1 to 1,000 mg in a suppository, per unit dosage form.

The amount of administration per day of a medicine in the above-mentioned dosage form varies depending on, for example, the symptoms, body weight, age, and gender of the patient, and are not specifically determined. However, the amount of administration per day for an adult (body weight 50 kg) is usually about 0.05 to 5,000 mg, and preferably 0.1 to 1,000 mg, and this is preferably administered once a day or dividedly in about 2 or 3 portions.

Since side effect of antitumor drug can be alleviated by using the agent for alleviating side effect of antitumor drug of the present invention, the amount of administration of the antitumor drug may be an amount of administration that is conventionally used, it is also possible to use the antitumor drug in an increased amount compared to the amount of administration conventionally used.

The proportions of administration or mixing of the agent for alleviating side effect of antitumor drug of the present invention and an antitumor drug are not particularly limited as long as the proportions are in the ranges that provide an augmented effect of the antitumor effect, the proportions may be such that the uracil compound represented by Formula (I) or a pharmacologically acceptable salt thereof is used in an amount of about 0.01 to 100 mol, and preferably about 0.07 to 64 mol, with respect to 1 mol of an antitumor drug. In the present invention, the proportion of administration or mixing of the antitumor drug is based on the amount of the active ingredient that provides an antitumor effect, as a

reference. For example, in the case of a medicine containing tegafur and gimeracil, the uracil compound represented by Formula (I) or a pharmacologically acceptable salt thereof may be used in an amount of about 0.01 to 100 mol, and preferably about 0.15 to 64 mol, with respect to 1 mol of tegafur, as the dosage per day. In the case of pemetrexed, the uracil compound represented by Formula (I) or a pharmacologically acceptable salt may be used in an amount of about 0.01 to 100 mol, and preferably about 0.07 to 12 mol, with respect to 1 mol of pemetrexed.

## EXAMPLES

Hereinafter, the present invention will be described in more detail by way of Examples and Test Examples; however, the present invention is not intended to be limited to embodiments described in these Examples.

### Example 1: Synthesis of (R)-N-(1-(3-(cyclopentyloxy)phenyl)ethyl)-3-((2,4-dioxo-3,4-dihydr opyrimidin-1(2H)-yl)methoxy)propane-1-sulfonamide

The compound was synthesized according to the method disclosed in Patent Document 1.
$^1$H-NMR (CDCl3) : δppm 1.53 (3H, d, J=6.8Hz), 1.56-1.98 (10H, m), 2.67-2.78 (1H, m), 2.80-2.91 (1H, m), 3.42-3.60 (2H, m), 4.51-4.63 (1H, m), 4.74-4.89 (2H, m), 5.05 (2H, s), 5.76 (1H, dd, J=7.8Hz, 2.2Hz), 6.77-6.89 (3H, m), 7.20-7.27 (2H, m), 8.76 (1H, brs) : LRMS (ESI) m/z 452[M + H]

### Example 2: Synthesis of N-(3-(cyclopropylmethoxy)benzyl)-3-((2,4-dioxo-3,4-dihydropyrimidi n-1(2H)--yl)methoxy)propane-1-sulfonamide

The compound was synthesized according to the method disclosed in Patent Document 1.
$^1$H-NMR (CDCl3) δ (ppm) : 0.30-0.39 (2H, m), 0.57-0.68 (2H, m), 1.20-1.31 (1H, m), 1.96-2.09 (2H, m), 3.0 (2H, t, J = 7.2Hz), 3.57-3.64 (2H, m), 3.81 (2H, d, J = 6.9Hz), 4.25 (2H, d, J = 6.1Hz), 4.89 (1H, brs), 5.09 (2H, s), 5.75 (1H, dd, J = 7.9, 1.8Hz), 6.76-6.90 (3H, m), 7.20-7.29 (2H, m), 8.90 (1H, brs)

### Example 3: Synthesis of (R)-N-(1-(3-(2,2,2-trifluoroethoxy)phenyl)ethyl)-3-((2,4-dioxo-3,4 -dihydropyrimidin-1(2H)-yl)methoxy)propane-1-sulfonamide

The compound was synthesized according to the method disclosed in Patent Document 1.
$^1$H-NMR (DMSO-D6) δ (ppm) : 1.37 (3H, d, J = 6.8Hz), 1.69-1.80 (2H, m), 2.58-2.70 (1H, m), 2.72-2.88 (1H, m), 3.31-3.46 (2H, m), 4.39-4.45 (1H, m), 4.69-4.79 (2H, m), 4.99 (2H, s), 5.60 (1H, dd, J = 8.1, 0.8Hz), 6.91-7.08 (3H, m), 7.26-7.31 (1H, m), 7.63 (1H, dd, J = 8.1, 0.8Hz), 7.73 (1H, d, J = 8.6Hz), 11.3 (1H, brs)

[Table 1]

| Compound of invention | $R^1$ | $R^2$ | $R^3$ |
|---|---|---|---|
| Compound 1 | $CH_3$ | H | 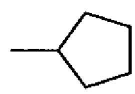 |
| Compound 2 | H | H | 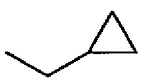 |
| Compound 3 | $CH_3$ | H | 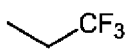 |

### Test Example 1: Toxicity alleviating effect against pemetrexed

Cells of a human lung cancer cell line, NCI-H441, were suspended in a PBS containing 50% MATRIGEL, and the suspension was transplanted subcutaneously into the right-side chest of each of 8-week old BALB/cA Jcl-nu mice (Crea-Japan, Inc.) in an amount of $5\times10^6$ cells/0.1 mL/body. At the time when the average tumor volume after grouping became more than 100 $mm^3$, the major axis and the minor axis of the tumor were measured, and grouping was performed such that there would be no fluctuations in the tumor volumes of the various groups (6 animals per group). Furthermore, the mice were maintained under conditions of low-folic acid feed breeding from the age of 5 weeks to the day of test completion.

Pemetrexed (manufactured by LC Laboratories, Inc.) was dissolved in physiological saline, and the solution was administered through the caudal vein on Day 1, which was the day after the day of grouping, and on Day 8, which was one week thereafter. Furthermore, Compound 1 was suspended in a 0.5% aqueous solution of hydroxypropylmethyl cellulose, and the suspension was orally administered once a day for 14 consecutive days from the day after the day of grouping. The amount of administration of pemetrexed was set to 50 mg/kg day, and the amount of administration of Compound 1 was set to 600 mg/kg/day.

After grouping, the body weights (BW) were measured twice a week, and the rate of body weight change (bodyweight change; BWC) was calculated by the following formula, the toxicity alleviating effect was evaluated.

$$\text{(Formula 1)}\quad BWC\ (\%) = [BW - (BW\ on\ Day\ 0)]/(BW\ on\ Day\ 0) \times 100$$

wherein BWC represents the rate of body weight change; BW represents the body weight on the day of measurement; and BW on Day 0 represents the body weight on the day of grouping.

The test results are presented in Fig. 1. In the case of a simple preparation of pemetrexed, remarkable reduction of the body weight was observed, whereas the body weight reduction was suppressed when the compound of the present invention was used in combination.

On Day 15 from the day of initiation of drug administration, the presence or absence of the occurrence of diarrhea was confirmed by observing the anal area of individual mice, and the proportion of mice recognized to show symptoms of diarrhea in each group was calculated as the diarrhea incidence (%), and the gastrointestinal toxicity alleviating effect was evaluated.

The test results are presented in Fig. 2. In the case of a simple preparation of pemetrexed, the occurrence of diarrhea was observed at a frequency of 60%, whereas the occurrence of diarrhea was not observed when the compound of the present invention was used in combination.

The numbers of deaths occurred during the test are shown in Table 2. In the case of a simple preparation of pemetrexed, one death case out of six animals was observed, whereas no death case was observed when the compound of the present invention was used in combination.

From the above results, it became clear that the compound of the present invention alleviates side effects of pemetrexed.

[Table 2]

| Drug | Dose (mg/kg/day) | No. of Death |
|---|---|---|
| Control | - | 0/6 |
| Pemetrexed | 50 | 1/6 |
| Pemetrexed + Compound 1 | 50 + 600 | 0/6 |

Test Example 2: Toxicity alleviating effect against S-1

Cells of a subcutaneously subcultured human breast cancer cell line, MX-1, were prepared into a fragment that measured 2 mm on each side, and the cell fragment was transplanted subcutaneously into the right-side chest of each of 5-week or 6-week old BALB/cA Jcl-nu mice (Crea-Japan, Inc.). At the time when the average tumor volume after grouping became more than 100 $mm^3$, the major axis and the minor axis of the tumor were measured, and grouping was performed such that there would be no fluctuations in the tumor volumes of the various groups (5 animals per group).

S-1 (manufactured by Taiho Pharmaceutical Co., Ltd.) and a test compound were suspended in a 0.5% aqueous solution of hydroxypropylmethyl cellulose, and the suspension was orally administered once a day for 14 consecutive days from the day after the day of grouping. The amount of administration of S-1 was set to 8.3 mg/kg/day, which was a dose expected to show effectiveness. The amount of administration of the test compound (Compound 2 or 3) was set to 75, 100, 150, 300, or 600 mg/kg/day, and the test compound was administered simultaneously with S-1.

After grouping, the body weights (BW) were measured twice a week, and the rate of body weight change (bodyweight change; BWC) was calculated by the following formula, and the toxicity alleviating effect was evaluated.

$$\text{(Formula 1)}\quad BWC\ (\%) = [BW - (BW\ on\ Day\ 0)]/(BW\ on\ Day\ 0) \times 100$$

wherein BWC represents the rate of body weight change; BW represents the body weight on the day of measurement;

and BW on Day 0 represents the body weight on the day of grouping.

The test results are shown in Fig. 3 and Fig. 4. In the case of a simple preparation of S-1, reduction of the body weight was observed, whereas the body weight reduction was suppressed when the compound of the present invention was used in combination.

Fifteen days after the day of initiation of drug administration, the presence or absence of the occurrence of diarrhea was checked by observing the anal area of individual mice, and the proportion of mice recognized to show symptoms of diarrhea in each group was calculated as the diarrhea incidence (%), and the gastrointestinal toxicity alleviating effect was evaluated.

The test results are shown in Fig. 5. In the case of a simple preparation of S-1, the occurrence of diarrhea was observed at a frequency of 80% or higher, whereas the occurrence of diarrhea was reduced in a dose-dependent manner when the compound of the present invention was used in combination.

The numbers of deaths occurred during the test are shown in Table 3. In the case of a simple preparation of S-1, one death case out of five animals was observed, whereas no death case was observed when the compound of the present invention was used in combination.

From the above results, it became clear that the compound of the present invention alleviates side effects of S-1.

[Table 3]

| Drug | Dose (mg/kg/day) | No. of Death |
|---|---|---|
| Control | - | 0/5 |
| S-1 | 8.3 | 1/5 |
| S-1 + Compound 2 | 8.3 + 100 | 0/5 |

Test Example 3: Toxicity alleviating effect against S-1

The body weights of 6-week old BALB/cA Jcl-nu mice (Crea-Japan, Inc.) were measured, and then grouping was performed by a random stratification method such that the body weight of each group after the grouping would be uniform (5 animals per group).

S-1 (manufactured by Taiho Pharmaceutical Co., Ltd.) and a test compound were suspended in a 0.5% aqueous solution of hydroxypropylmethyl cellulose, and the suspension was orally administered once a day for 14 consecutive days from the day after the day of grouping. The amount of administration of S-1 was set to 12 mg/kg/day, which was a toxic dose. The amount of administration of the test compound (Compound 1) was set to 300 or 600 mg/kg/day, and the test compound was administered simultaneously with S-1.

After grouping, the body weights (BW) were measured twice a week, and the rate of body weight change (body weight change; BWC) was calculated by the following formula, and the toxicity alleviating effect was evaluated.

$$\text{(Formula 1)} \quad \text{BWC (\%)} = [\text{BW} - (\text{BW on Day 0})]/(\text{BW on Day 0}) \times 100$$

wherein BWC represents the rate of body weight change; BW represents the body weight on the day of measurement; and BW on Day 0 represents the body weight on the day of grouping.

The test results are shown in Fig. 6. In the case of a simple preparation of S-1, reduction of the body weight was observed (as shown in Table 3, the body weight reduction was noticeable in two examples out of five examples, and since the animals died before or after Day 10, the average weight obtained by body weight measurement after Day 12 appears to increase). When the compound of the present invention was used in combination, the body weight reduction was suppressed in a dose-dependent manner.

Furthermore, the presence or absence of the occurrence of diarrhea was checked for 14 consecutive days from the day of initiation of drug administration, by observing the anal area of individual mice, and the proportion of mice recognized to show symptoms of diarrhea in each group was calculated as the diarrhea incidence (%), and the gastrointestinal toxicity alleviating effect was evaluated.

The test results are shown in Fig. 7. In the case of a simple preparation of S-1, the animals were found to have diarrhea at a high frequency during the administration period, and particularly from Day 8 to Day 10, the occurrence of diarrhea was observed at a frequency of 100%. On the other hand, the occurrence of diarrhea was reduced in a dose-dependent manner when the compound of the present invention was used in combination.

Furthermore, the numbers of deaths occurred during the test are shown in Table 4. In the case of a simple preparation of S-1, two death cases out of five animals were observed, whereas the number of death cases decreased

in a dose-dependent manner when the compound of the present invention was used in combination. From the above results, it became clear that the compound of the present invention alleviates side effects of S-1.

[Table 4]

| Drug | Dose (mg/kg/day) | No. of Death |
|---|---|---|
| Control | - | 0/5 |
| S-1 | 12 | 2/5 |
| S-1 + Compound 1 | 12 + 300 | 1/5 |
| S-1 + Compound 1 | 12 + 600 | 0/5 |

Test Example 4: Toxicity alleviating effect against S-1

A plurality of clinical tests of administering S-1 alone to human tumor patients, and a plurality of clinical tests of administering S-1 and Compound 1 together to human tumor patients were carried out.

The severity of diarrhea manifested by the patients during the period from the initiation of the clinical tests to the termination of the tests based on the abort criteria (aggravation of the original disease, and withdrawal of consent by the patient), was evaluated using CTCAE.

In the clinical tests, in principle, S-1 was administered after meal at a dose of 30 $mg/m^2$. However, in regard to the clinical tests of administering S-1 alone, a plurality of tests of administering S-1 after meal at a dose of 36 $mg/m^2$ was performed, in addition to three tests of administering S-1 at a dose of 30 $mg/m^2$. The diarrhea incidence was determined as an overall result of these tests (the total number N is 1,800). Furthermore, in the case of administering at a dose of 36 $mg/m^2$, the $AUC_{0-12}$ value of 5-FU is almost equal to that in the case of administering after meal at a dose of 30 $mg/m^2$.

Compound 1 was administered simultaneously with S-1.

A comparison was made between the frequencies of occurrence of diarrhea for all grades (All) and for Grade 3 (G3) or higher in patients administered with a simple preparation of S-1 and with S-1 and Compound 1 together, and the results are shown in Table 5.

The diarrhea incidence for all grades and the diarrhea incidence for Grade 3 or higher in patients administered with a simple preparation of S-1 were 29.6% to 49% and 3.6% to 21%, respectively, while the same diarrhea incidences in patients administered with S-1 and Compound 1 in combination were 18.8% and 19%, as well as 0% and 1.6%, respectively. Thus, the frequency of occurrence of diarrhea was decreased by using the compound of the present invention in combination.

From the above results, it became clear that the compound of the present invention alleviates side effect of S-1.

[Table 5]

| S-1 | | | | | | | | S-1 + Compound 1 | | | |
|---|---|---|---|---|---|---|---|---|---|---|---|
| 30 $mg/m^2$ (N = 57) | | 30 $mg/m^2$ (N = 27) | | 30 $mg/m^2$ (N = 45) | | 36 $mg/m^2$ (N = 1800) | | 30 $mg/m^2$ (N = 64) | | 30 $mg/m^2$ (N = 37) | |
| All (%) | ≥ G3 (%) | All (%) | ≥ G3 (%) | All (%) | ≥ G3 (%) | All (%) | ≥ G3 (%) | All (%) | ≥ G3 (%) | All (%) | ≥ G3 (%) |
| 49 | 21 | 29.6 | 3.7 | 44.4 | 20 | 31.7 | 3.6 | 18.8 | 1.6 | 19 | 0 |

Test Example 5: Toxicity alleviating effect against FCD

The toxicity alleviating effect was investigated using FCD (tegafur/gimeracil = 1 : 0.4 (molar ratio)), which was obtained by excluding oteracil that contributes to gastrointestinal alleviation (Jpn J Clin Oncol 2009:39(1) 2-15) from among tegafur, gimeracil, and oteracil, which are the ingredients of S-1.

The body weights of 6-week old BALB/cA Jcl-nu mice (Crea-Japan, Inc) were measured, and then grouping was performed by a random stratification method such that the body weight of each group after the grouping would be uniform (5 animals per group).

FCDwas suspended in a 0.5% aqueous solution of hydroxypropylmethyl cellulose, and the suspension was orally administered once a day for 14 consecutive days from the day after the day of grouping. The amount of administration of FCD was set to 10 mg/kg/day in terms of tegafur. The amount of administration of Compound 1 was set to 150, 300,

or 600 mg/kg/day, and Compound 1 was administered simultaneously with FCD.

After grouping, the body weights (BW) were measured twice a week, and the rate of bodyweight change (bodyweight change; BWC) was calculated by the following formula, and the toxicity alleviating effect was evaluated.

$$\text{(Formula 1)} \quad \text{BWC (\%)} = [\text{BW} - (\text{BW on Day 0})]/(\text{BW on Day 0}) \times 100$$

wherein BWC represents the rate of body weight change; BW represents the body weight on the day of measurement; and BW on Day 0 represents the body weight on the day of grouping.

The test results are shown in Fig. 8. Reduction of the body weight was observed in the case of a simple preparation of FCD, whereas the body weight reduction was suppressed when the compound of the present invention was used in combination.

Furthermore, the presence or absence of the occurrence of diarrhea was checked for 14 consecutive days from the day of initiation of drug administration, by observing the anal area of individual mice, and the proportion of mice recognized to show symptoms of diarrhea in each group was calculated as the diarrhea incidence (%), and the gastrointestinal toxicity alleviating effect was evaluated.

The test results are shown in Fig. 9.

In the case of a simple preparation of FCD, the animals were found to have diarrhea at a high frequency during the administration period, and particularly on Day 10, the occurrence of diarrhea was observed at a frequency of 100%. On the other hand, the occurrence of diarrhea was reduced in a dose-dependent manner when the compound of the present invention was used in combination.

From the above results, it became clear that the compound of the present invention alleviates side effects of a medicine containing tegafur and gimeracil as active ingredients.

## Claims

1. An agent for alleviating side effect of antitumor drug, the agent comprising a uracil compound represented by the following Formula (I) or a pharmacologically acceptable salt thereof as an active ingredient:

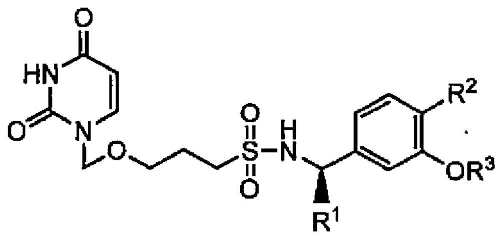

(I)

wherein $R^1$ represents a hydrogen atom or a C1-6 alkyl group; $R^2$ represents a hydrogen atom or a halogen atom; and $R^3$ represents a C1-6 alkyl group, a C2-6 alkenyl group, a C3-6 cycloalkyl group, a (C3-6 cycloalkyl) -C1-6 alkyl group, a halogeno-C1-6 alkyl group, or a saturated heterocyclic group.

2. The agent for alleviating side effect of antitumor drug according to claim 1, wherein the uracil compound is a compound represented by Formula (I), in which $R^1$ represents a hydrogen atom, a methyl group, or an ethyl group; $R^2$ represents a hydrogen atom; and $R^3$ represents a cyclopentyl group, a cyclopropylmethyl group, or a 2,2,2-trifluoroethyl group.

3. The agent for alleviating side effect of antitumor drug according to claim 1, wherein the uracil compound is selected from the group consisting of the following compounds:

N-(1-(3-(cyclopentyloxy)phenyl)ethyl)-3-((2,4-dioxo-3,4-dihydropyr imidin-1(2H)-yl)methoxy)propane-1-sulfonamide,
N-(3-(cyclopropylmethoxy)benzyl)-3-((2,4-dioxo-3,4-dihydropyrimidi n-1(2H)-yl)methoxy)propane-1-sulfonamide, and
(R)-N--(1-(3-(2,2,2-trifluoroethoxy)phenyl)ethyl)-3-((2,4-dioxo-3,4 -dihydropyrimidin-1(2H=-yl)methoxy)propane-1-sulfonamide.

4. The agent for alleviating side effect of antitumor drug according to anyone of claims 1 to 3, wherein the antitumor drug is anantimetabolite.

5. The agent for alleviating side effect of antitumor drug according to any one of claims 1 to 3 , wherein the antitumor drug is any one selected from 5-fluorouracil (5-FU), a tegafur/gimeracil/oteracil potassium combination drug (tegafur : gimeracil : oteracil = 1 : 0.4 : 1 (molar ratio)), and pemetrexed.

6. Use of a uracil compound represented by the following Formula (I) or a pharmacologically acceptable salt thereof, for producing an agent for alleviating side effect of antitumor drug:

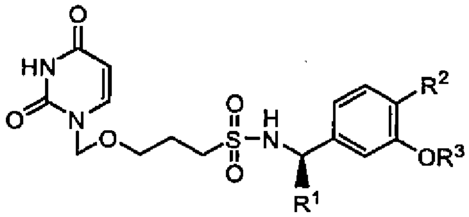

(I)

wherein $R^1$ represents a hydrogen atom or a C1-6 alkyl group; $R^2$ represents a hydrogen atom or a halogen atom; and $R^3$ represents a C1-6 alkyl group, a C2-6 alkenyl group, a C3-6 cycloalkyl group, a (C3-6 cycloalkyl) -C1-6 alkyl group, a halogeno-C1-6 alkyl group, or a saturated heterocyclic group.

7. The use according to claim 6, wherein the uracil compound is a compound represented by Formula (I), in which $R^1$ represents a hydrogen atom, a methyl group, or an ethyl group; $R^2$ represents a hydrogen atom; and $R^3$ represents a cyclopentyl group, a cyclopropylmethyl group, or a 2,2,2-trifluoroethyl group.

8. The use according to claim 6, wherein the uracil compound is a compound selected from the group consisting of the following compounds:

N-(1-(3-(cyclopentyloxy)phenyl)ethyl)-3-((2,4-dioxo-3,4-dihydropyr imidin-1(2H)-yl) methoxy)propane-1--sulfonamide,
N-(3-(cyclopropylmethoxy)benzyl)-3-((2,4-dioxo-3,4-dihydropyrimidi n-1(2H)-yl)methoxy)propane-1-sulfonamide, and
(R)-N-(1-(3-(2,2,2-trifluoroethoxy)phenyl)ethyl)-3-((2,4-dioxo-3,4 -dihydropyrimidin-1(2H)-yl)methoxy)propane-1-sulfonamide.

9. The use according to any one of claims 6 to 8 , wherein the antitumor drug is an antimetabolite.

10. The use according to any one of claims 6 to 8, wherein the antitumor drug is any one selected from 5-fluorouracil (5-FU), a tegafur/gimeracil/oteracil potassium combination drug (tegafur : gimeracil : oteracil = 1 : 0.4 : 1 (molar ratio)), and pemetrexed.

11. A uracil compound represented by the following Formula (I) or a pharmacologically acceptable salt thereof, for use in the alleviation of side effect of antitumor drug:

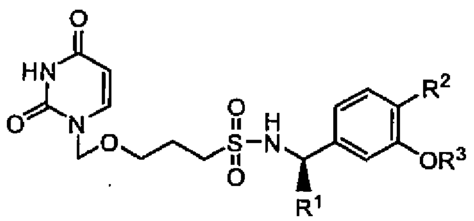

(I)

wherein $R^1$ represents a hydrogen atom or a C1-6 alkyl group; $R^2$ represents a hydrogen atom or a halogen atom; and $R^3$ represents a C1-6 alkyl group, a C2-6 alkenyl group, a C3-6 cycloalkyl group, a (C3-6 cycloalkyl) -C1-6 alkyl group, a halogeno-Cl-6 alkyl group, or a saturated heterocyclic group.

12. The uracil compound or a pharmacologically acceptable salt thereof according to claim 11, wherein in Formula (I), $R^1$ represents a hydrogen atom, a methyl group, or an ethyl group; $R^2$ represents a hydrogen atom; and $R^3$ represents a cyclopentyl group, a cyclopropylmethyl group, or a 2,2,2-trifluoroethyl group.

13. The uracil compound or a pharmacologically acceptable salt thereof according to claim 11, wherein the compound or the salt is a compound selected from the group consisting of the following compounds, or a pharmacologically acceptable salt thereof:

N-(1-(3-(cyclopentyloxy)phenyl)ethyl)-3-((2,4-dioxo-3,4-dihydropyr imidin-1(2H)-yl)methoxy)propane-1-sulfonamide,
N-(3-(cyclopropylmethoxy)benzyl)-3-((2,4-dioxo-3,4-dihydropyrimidi n-1(2H)-yl)methoxy)propane-1-sulfonamide, and
(R)-N-(1-(3-(2,2,2-trifluoroethoxy)phenyl)ethyl)-3-((2,4-dioxo-3,4 -dihydropyrimidin-1(2H)-yl)methoxy)propane-1-sulfonamide.

14. The uracil compound or a pharmacologically acceptable salt thereof according to any one of claims 11 to 13, wherein the antitumor drug is an antimetabolite.

15. The uracil compound or a pharmacologically acceptable salt thereof according to any one of claims 11 to 13, wherein the antitumor drug is any one selected from 5-fluorouracil (5-FU), a tegafur/gimeracil/oteracil potassium combination drug (tegafur : gimeracil : oteracil = 1 : 0.4 : 1 (molar ratio)), and pemetrexed.

16. A method for alleviating side effect of antitumor drug, the method comprising administering an effective amount of a uracil compound represented by the following Formula (I) or a pharmacologically acceptable salt thereof to an object in need of the alleviation:

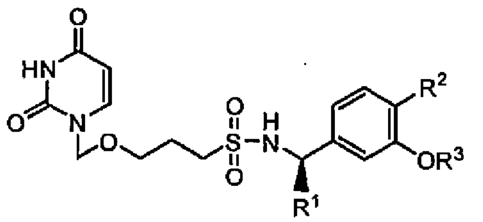
(I)

wherein $R^1$ represents a hydrogen atom or a C1-6 alkyl group; $R^2$ represents a hydrogen atom or a halogen atom; and $R^3$ represents a C1-6 alkyl group, a C2-6 alkenyl group, a C3-6 cycloalkyl group, a (C3-6 cycloalkyl) -C1-6 alkyl group, a halogeno-C1-6 alkyl group, or a saturated heterocyclic group.

17. The method according to claim 16, wherein the uracil compound is a compound represented by Formula (I), in which $R^1$ represents a hydrogen atom, a methyl group, or an ethyl group; $R^2$ represents a hydrogen atom; and $R^3$ represents a cyclopentyl group, a cyclopropylmethyl group, or a 2,2,2-trifluoroethyl group.

18. The method according to claim 16, wherein the uracil compound is selected from the group consisting of the following compounds:

N-(1-(3-(cyclopentyloxy)phenyl)ethyl)-3-((2,4-dioxo-3,4-dihydropyr imidin-1(2H)-yl)methoxy)propane-1-sulfonamide,
N-(3-(cyclopropylmethoxy)benzyl)-3-((2,4-dioxo-3,4-dihydropyrimidi n-1(2H)-yl)methoxy)propane-1-sulfonamide, and
(R)-N-(1-(3-(2,2,2-trifluoroethoxy)phenyl)ethyl)-3-((2,4-dioxo-3,4 -dihydropyrimidin-1(2H)-yl)methoxy)propane-1-sulfonamide.

19. The method according to any one of claims 16 to 18, wherein the antitumor drug is an antimetabolite.

20. The method according to any one of claims 16 to 18, wherein the antitumor drug is any one selected from 5-fluorouracil (5-FU), a tegafur/gimeracil/oteracil potassium combination drug (tegafur : gimeracil : oteracil = 1 : 0.4 : 1 (molar ratio)), and pemetrexed.

Figure 1

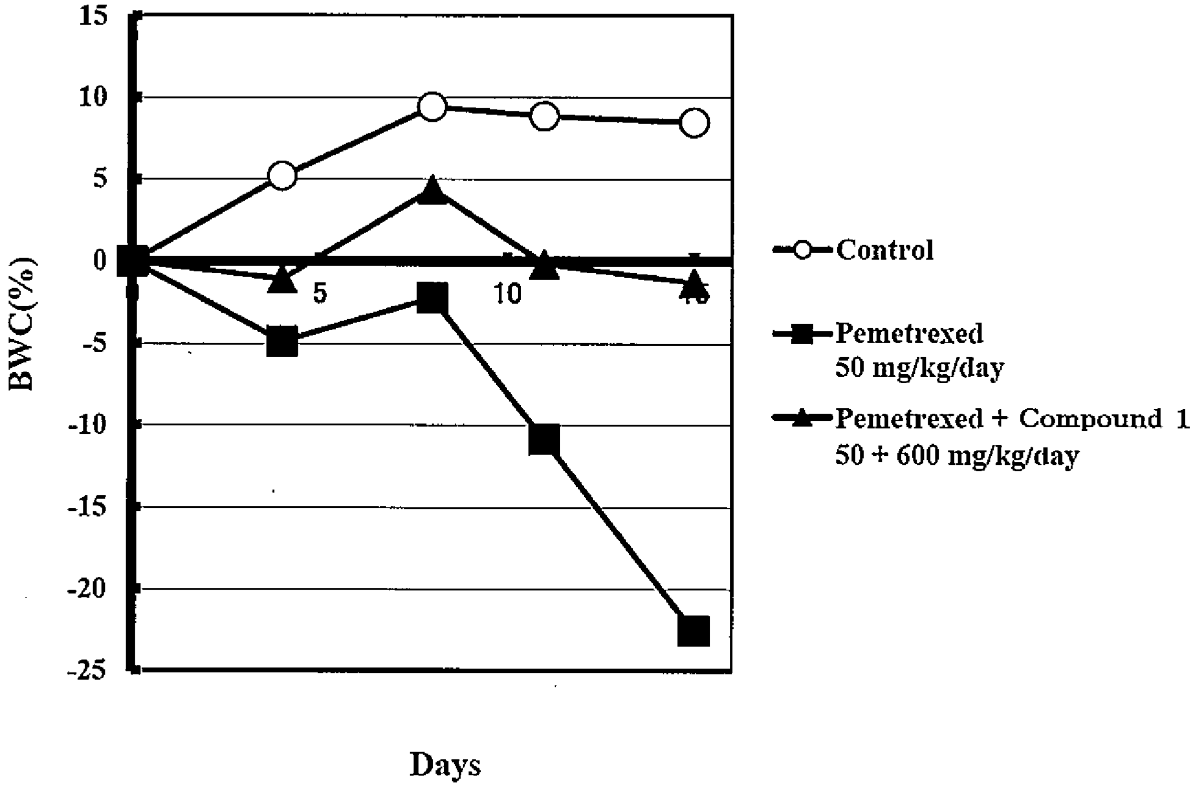
15
10
5
0
-5
-10
-15
-20
-25
BWC(%)
5
10
Days
Control
Pemetrexed
50 mg/kg/day
Pemetrexed + Compound 1
50 + 600 mg/kg/day

Figure 2

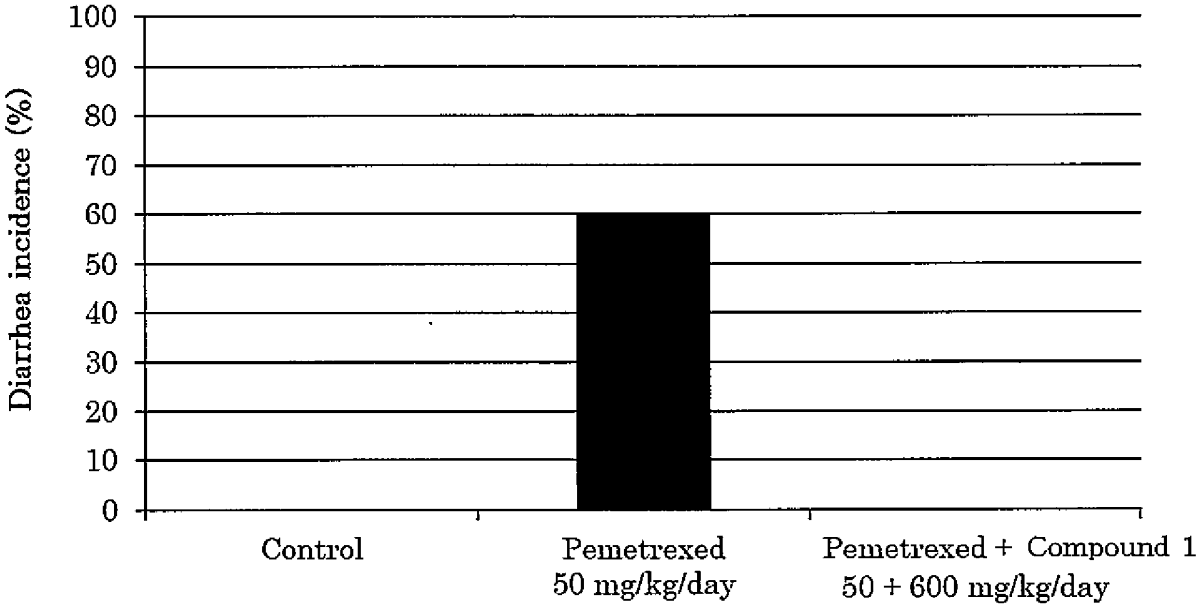
100
90
80
70
60
50
40
30
20
10
0
Diarrhea incidence (%)
Control
Pemetrexed
50 mg/kg/day
Pemetrexed + Compound 1
50 + 600 mg/kg/day

Figure 3

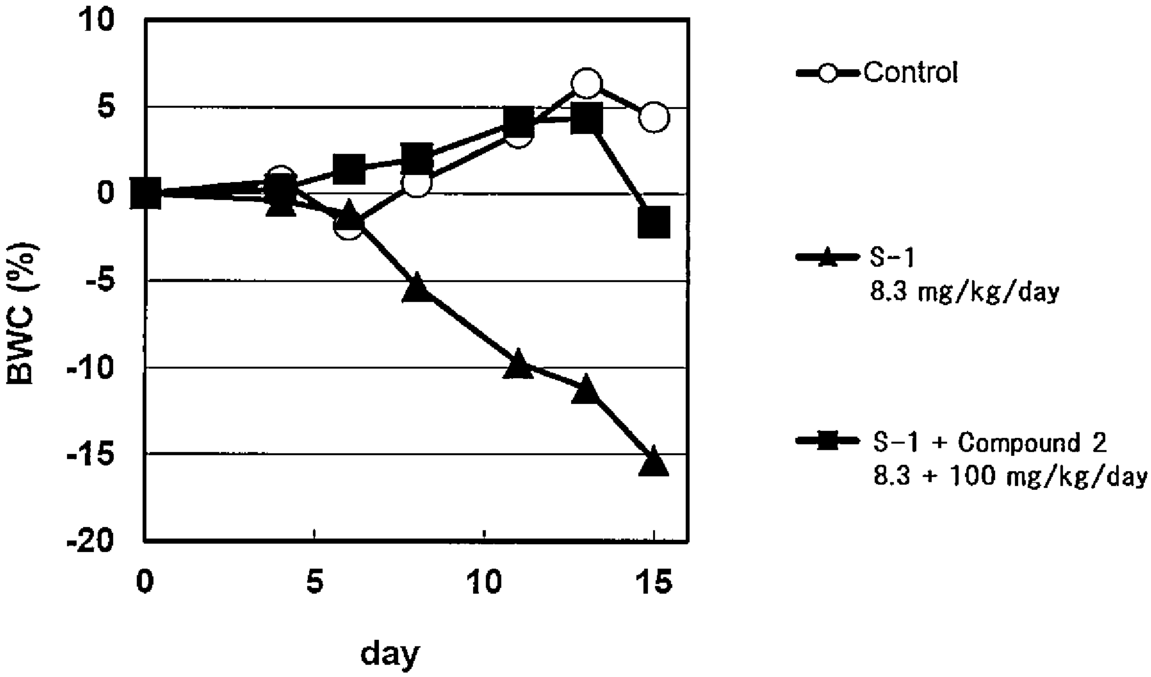
10
5
0
-5
-10
-15
-20
BWC (%)
0
5
10
15
day
Control
S-1
8.3 mg/kg/day
S-1 + Compound 2
8.3 + 100 mg/kg/day

Figure 4

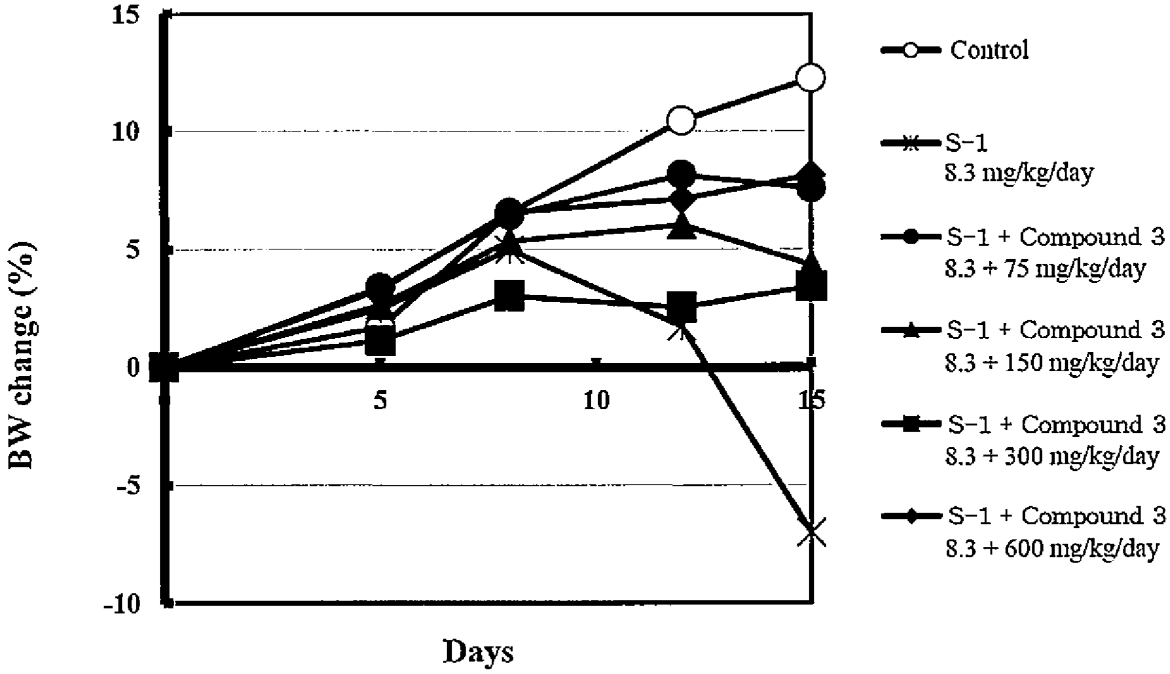
15
10
5
0
-5
-10
BW change (%)
5
10
15
Days
Control
S-1
8.3 mg/kg/day
S-1 + Compound 3
8.3 + 75 mg/kg/day
S-1 + Compound 3
8.3 + 150 mg/kg/day
S-1 + Compound 3
8.3 + 300 mg/kg/day
S-1 + Compound 3
8.3 + 600 mg/kg/day

Figure 5

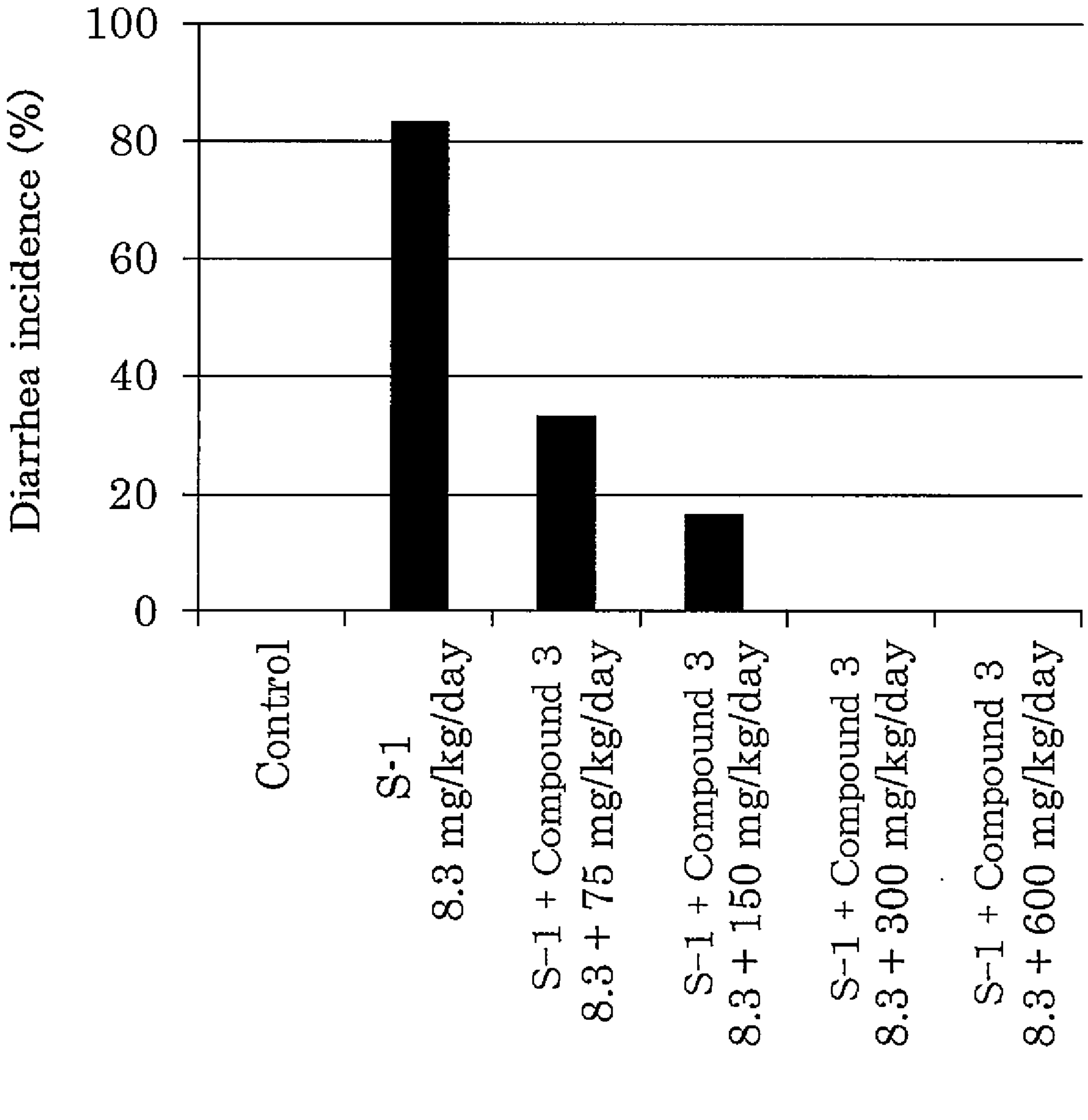
Diarrhea incidence (%)
100
80
60
40
20
0
Control
S-1
8.3 mg/kg/day
S-1 + Compound 3
8.3 + 75 mg/kg/day
S-1 + Compound 3
8.3 + 150 mg/kg/day
S-1 + Compound 3
8.3 + 300 mg/kg/day
S-1 + Compound 3
8.3 + 600 mg/kg/day

Figure 6

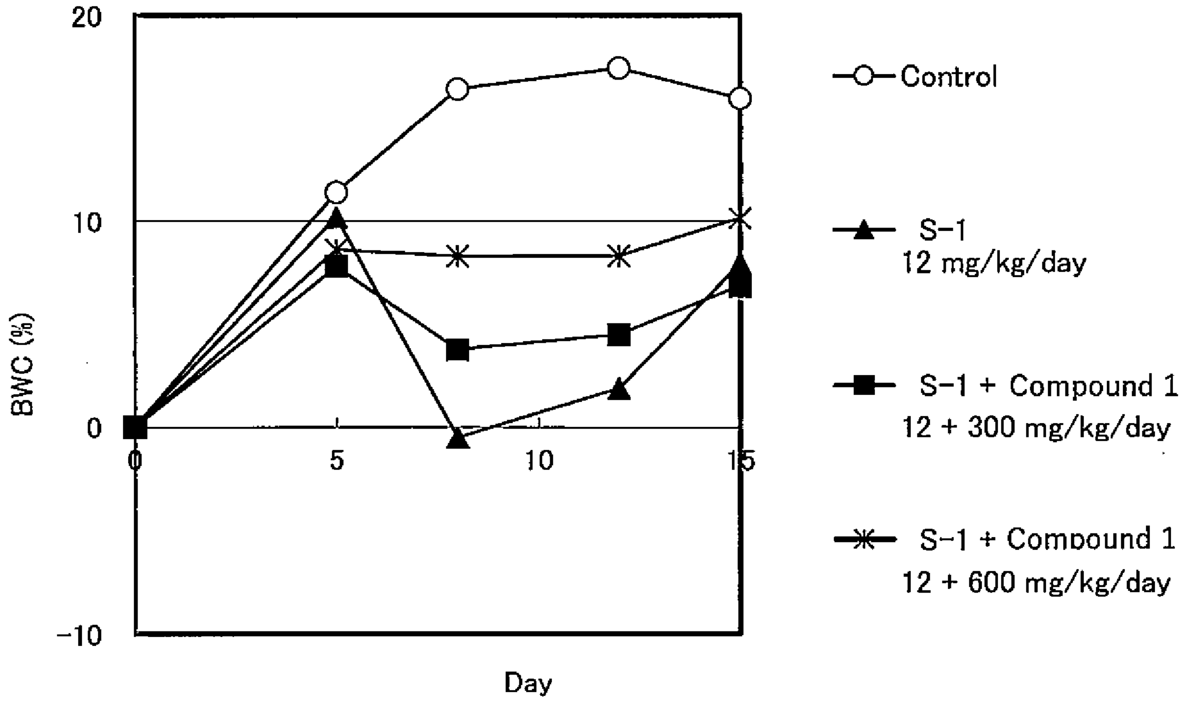
20
10
0
-10
BWC (%)
0
5
10
15
Day
Control
S-1
12 mg/kg/day
S-1 + Compound 1
12 + 300 mg/kg/day
S-1 + Compound 1
12 + 600 mg/kg/day

Figure 7

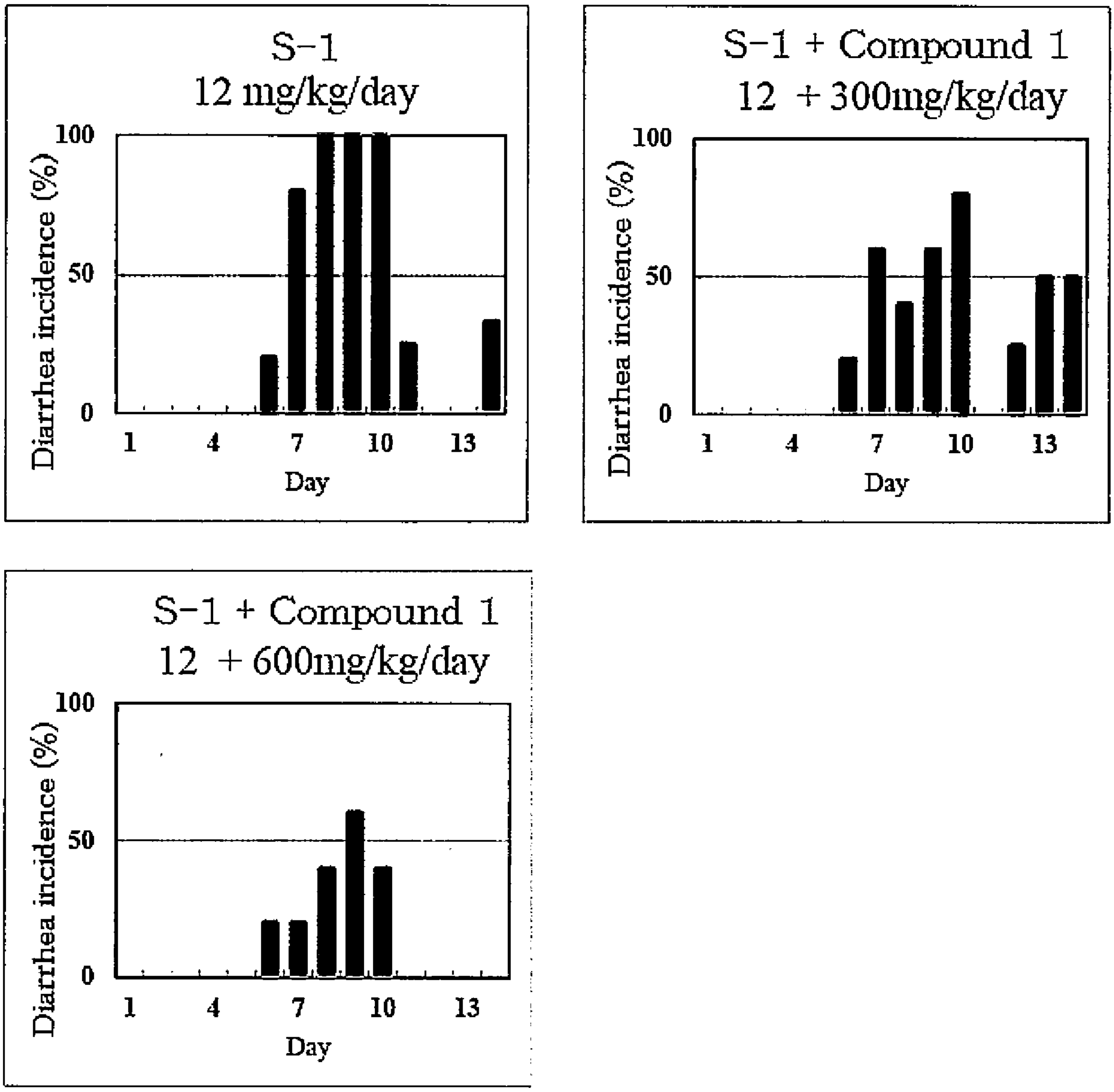
S-1
12 mg/kg/day
S-1 + Compound 1
12 + 300mg/kg/day
S-1 + Compound 1
12 + 600mg/kg/day
Diarrhea incidence (%)
100
50
0
1
4
7
10
13
Day

Figure 8

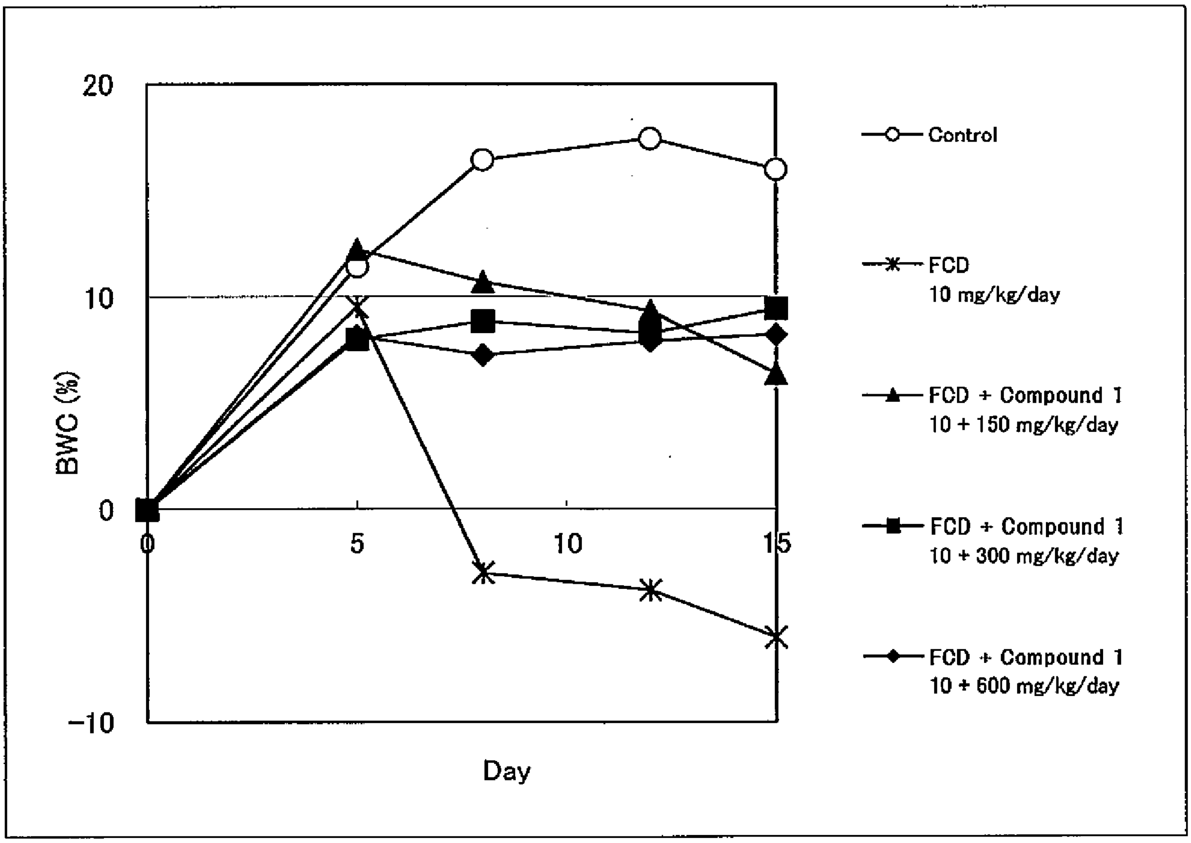
20
10
0
-10
BWC (%)
0
5
10
15
Day
Control
FCD
10 mg/kg/day
FCD + Compound 1
10 + 150 mg/kg/day
FCD + Compound 1
10 + 300 mg/kg/day
FCD + Compound 1
10 + 600 mg/kg/day

Figure 9

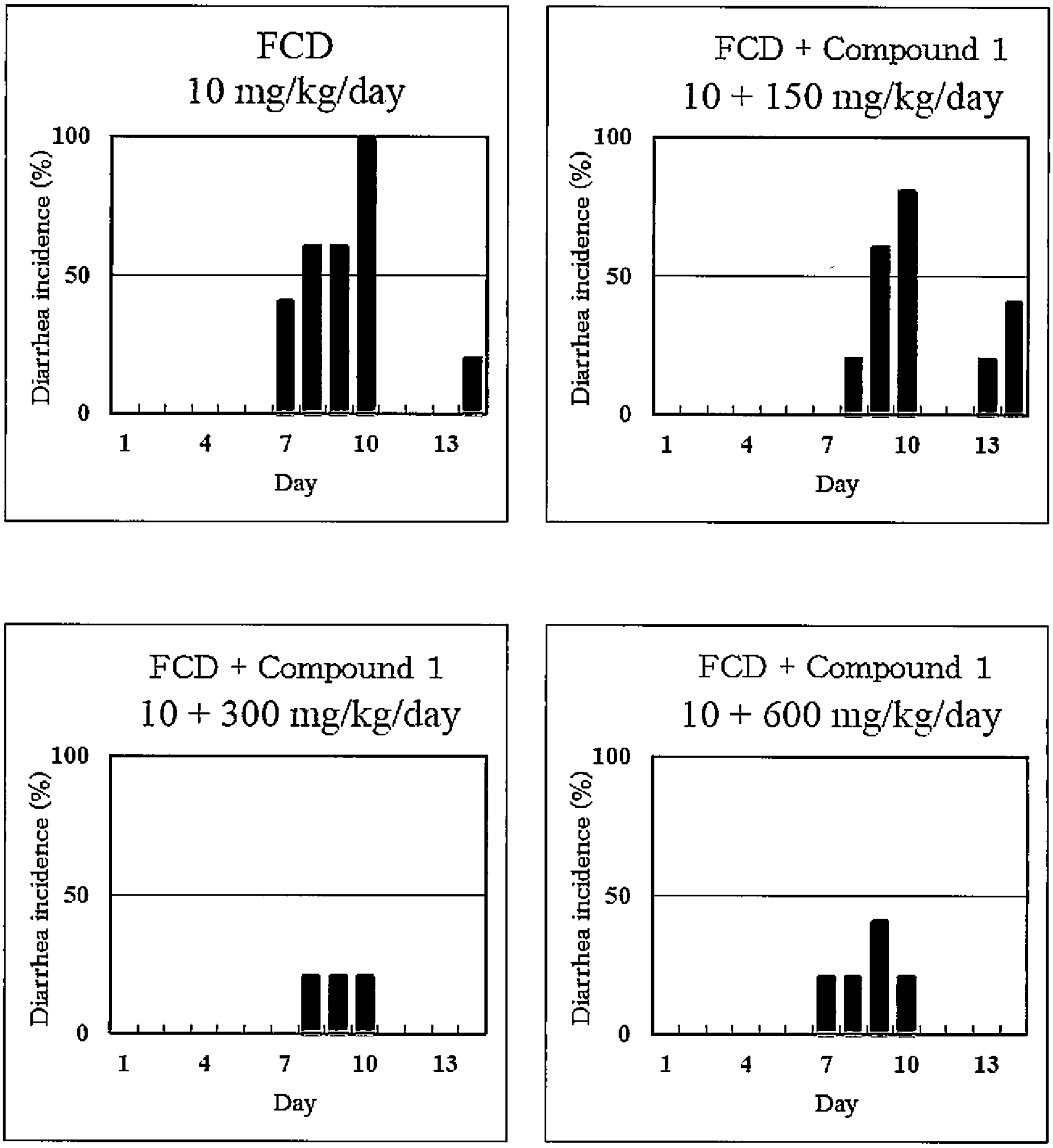
FCD
10 mg/kg/day
FCD + Compound 1
10 + 150 mg/kg/day
FCD + Compound 1
10 + 300 mg/kg/day
FCD + Compound 1
10 + 600 mg/kg/day
Diarrhea incidence (%)
100
50
0
1
4
7
10
13
Day

## INTERNATIONAL SEARCH REPORT

International application No. PCT/JP2016/063494

A. CLASSIFICATION OF SUBJECT MATTER
*A61K31/513*(2006.01)i, *A61P1/00*(2006.01)i, *A61P43/00*(2006.01)i, *A61K31/4412*(2006.01)n, *A61K31/519*(2006.01)n, *A61K31/53*(2006.01)n, *A61P35/00*(2006.01)n

According to International Patent Classification (IPC) or to both national classification and IPC

B. FIELDS SEARCHED

Minimum documentation searched (classification system followed by classification symbols)
A61K31/513, A61P1/00, A61P43/00, A61K31/4412, A61K31/519, A61K31/53, A61P35/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)
JSTPlus/JMEDPlus/JST7580(JDreamIII), CAplus/REGISTRY/MEDLINE/EMBASE/BIOSIS(STN)

C. DOCUMENTS CONSIDERED TO BE RELEVANT

| Category* | Citation of document, with indication, where appropriate, of the relevant passages | Relevant to claim No. |
|---|---|---|
| X<br>A | WO 2011/065541 A1 (Taiho Pharmaceutical Co., Ltd.),<br>03 June 2011 (03.06.2011),<br>claims 1 to 4; examples 1 to 2, 10; test example 9; fig. 5 to 7<br>& US 2012/0225838 A1<br>claims 1 to 4; examples 1 to 2, 10; test example 9; fig. 5 to 7<br>& JP 5336606 B & WO 2011/065541 A1<br>& EP 2508185 A1 & AU 2010323454 A<br>& CA 2782280 A & CN 102612369 A<br>& MX 2012006183 A & RU 2012127309 A<br>& HK 1171185 A & TW 201124387 A<br>& KR 10-2012-0116925 A & CN 103948599 A | 11-15<br>1-10,16-20 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>14 July 2016 (14.07.16) | Date of mailing of the international search report<br>26 July 2016 (26.07.16) |
| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (January 2015)

**INTERNATIONAL SEARCH REPORT**

International application No. PCT/JP2016/063494

C (Continuation). DOCUMENTS CONSIDERED TO BE RELEVANT

| Category* | Citation of document, with indication, where appropriate, of the relevant passages | Relevant to claim No. |
|---|---|---|
| X<br>A | MIYAHARA, S. et al, Discovery of a novel class of potent human deoxyuridine triphosphatase inhibitors remarkably enhancing the antitumor activity of thymidylate synthase inhibitors, J. Med. Chem., 2012, Vol.55, No.7, pp.2970-2980, ISSN 0022-2623 ABSTRACT, Figure 7. | 11,14-15<br>1-10,12-13, 16-20 |
| X<br>A | WO 2009/147843 A1 (Taiho Pharmaceutical Co., Ltd.),<br>10 December 2009 (10.12.2009),<br>claim 1; examples 1, 5, 53<br>& US 2011/0082163 A1<br>claim 1; examples 1, 5, 53<br>& JP 4688975 B & WO 2009/147843 A1<br>& EP 2295414 A1 & AU 2009254656 A<br>& CA 2726579 A & CN 102056905 A<br>& RU 2010154425 A & HK 1154385 A<br>& KR 10-2011-0013427 A & TW 201000452 A<br>& MY 149180 A | 11-15<br>1-10,16-20 |
| A | UTSUGI, T., New challenges and inspired answers for anticancer drug discovery and development, Jpn. J. Clin. Oncol., 2013, Vol.43, No.10, pp.945-953, ISSN 0368-2811 Figure 3. | 1-20 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009147843 A
- WO 2011065541 A


### Non-patent literature cited in the description


- *J Clin Pathol,* April 2009, vol. 62 (4), 364-9
- *Int J Cancer,* 22 December 1999, vol. 84 (6), 614-7
- *Cancer Res,* 01 July 2000, vol. 60 (13), 3493-503
- *Mol Pharmacol,* September 2004, vol. 66 (3), 620-6
- *Jpn J Clin Oncol,* 2009, vol. 39 (1), 2-15